# EUROPÄISCHE PATENTSCHRIFT

(11) **EP 0 542 039 B1**
(45) Veröffentlichungstag und Bekanntmachung des Hinweises auf die Patenterteilung: **26.06.1996**
(21) Anmeldenummer: 92118401.6
(22) Anmeldetag: 28.10.1992
(51) Int. Cl.: C07D 295/02

(54) **Verfahren zur Herstellung von Aminen durch reduktive Aminierung**
Process for the preparation of aminos through reductive amination
Procédé de préparation d'amines par amination réductrice

(30) Priorität: 11.11.1991 DE 4137013
(43) Veröffentlichungstag der Anmeldung: 19.05.1993
(73) Patentinhaber: BASF Aktiengesellschaft, D-67063 Ludwigshafen (DE)
(72) Erfinder: Dingerdissen, Uwe, Dr., W-6104 Seeheim-Jugenheim 1 (DE); Hoelderich, Wolfgang, Prof. Dr., W-6710 Frankenthal (DE)

(56) Entgegenhaltungen:
- WO-A-91/10641
- DE-A- 3 116 395
- US-A- 4 806 690
- DATABASE WPIL, Week 8815, Derwent Publications Ltd., London, GB; AN 88-101677

## Beschreibung

Die vorliegende Erfindung betrifft ein Verfahren zur Herstellung von Amin durch reduktive Aminierung von Ketonen.

Die Herstellung von tert. Aminen durch reduktive Aminierung ist aus Organic Reactions, Vol. 4, Kapitel 3, Seite 174-256 (1948), oder Houben-Weyl, Methoden der organischen Chemie, 4. Aufl. G. Thieme Verlag Stuttgart, Bd 11/1 (1957), S. 611 bis 663 bekannt. Die reduktive Aminierung wird durch Umsetzung des Ketons oder Aldehyds mit dem Amin und Wasserstoff in Gegenwart eines metallhaltigen Katalysators durchgeführt. Bei der metallischen Komponente des Katalysators kann es sich um z.B. Nickel oder Platin oder Palladium handeln. Besonders herausgestellt wird bei Organic Reactions, daß die Reaktion von Ketonen mit niederem Molekulargewicht mit Aminen mit niederem Molekulargewicht Platin als Katalysator am geeignetsten ist. Bei der Umsetzung von sekundären Aminen mit Ketonen am Platin-Katalysator werden danach Ausbeuten von bis zu 47 % der Theorie erzielt.

In DE-A 25 35 725 wird in der Flüssigphase an einem Nickel-Trägerkatalysator reduktiv aminiert. Hier werden im Mitteldruckbereich bis zu 96 % Aminausbeuten erzielt. Das Verfahren wird in der Flüssigphase durchgeführt und zeichnet sich durch sehr lange Verweilzeiten (3 bis 10 h) und einer daraus resultierende geringe Raum-Zeit-Ausbeute aus.

Die bisher bekannten Verfahren haben den Nachteil, daß entweder geringe Ausbeuten und/oder lange Verweilzeiten in Kauf genommen werden mußten.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminen der allgemeinen Formel I gefunden 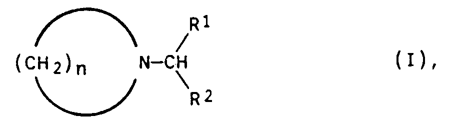
in der
- R¹, R²: C₁- bis C₂₀-Alkyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl und heterocyclische Reste und
- n: eine ganze Zahl von 3 bis 7
bedeuten, dadurch gekennzeichnet, daß man Ketone der allgemeinen Formel II 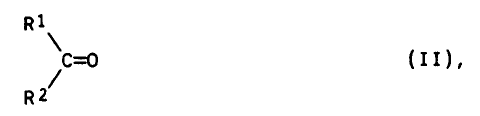
in der R¹ und R² oben genannten Bedeutung haben, mit einem cyclischen Amin der allgemeinen Formel III 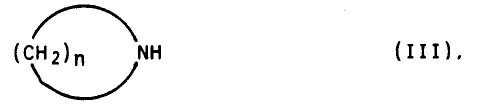
in der n die oben genannten Bedeutungen hat, mit Wasserstoff in Gegenwart von Zeolithen und/oder SiO₂ mit Zeolithstruktur und/oder Phosphaten und/oder Phosphaten mit Zeolithstruktur als Katalysatoren bei Temperaturen von 50 bis 500°C und Drücken von 0,01 bis 50 bar umsetzt.

Das erfindungsgemäße Verfahren zur Herstellung von Aminen I kann wie folgt durchgeführt werden:
Die Umsetzung erfolgt durch Kontakt einer Mischung aus einem Keton II und einem Amin III mit Wasserstoff an Zeolithen und/oder SiO₂ mit Zeolithstruktur und/oder Phosphaten und/oder Phosphaten mit Zeolithstruktur als Katalysatoren bei Temperaturen von 50 bis 500°C und Drücken von 0,01 bis 50 bar.

Die Reaktion kann sowohl in der Flüssigphase (Suspension-, Riesel- oder Sumpffahrweise) bei Temperaturen von 50 bis 200°C und Drücken von 0,05 bis 5 bar, bevorzugt in der Gasphase bei Temperaturen von 100 bis 500°C, bevorzugt 200 bis 400°C und Drücken von 0,01 bis 50 bar, bevorzugt 0,1 bis 30 bar, besonders bevorzugt 0,5 bis 5 bar diskontinuierlich, bevorzugt kontinuierlich durchgeführt werden. Die Katalysatorbelastung sollte in aller Regel bei WHSV = 0,1 bis 20 h⁻¹, bevorzugt 0,5 bis 5 h⁻¹ (g Einsatzgemisch/g Katalysator und Stunde) liegen.

Das Verfahren wird in der Regel bei Normaldruck (Atmosphärendruck) oder je nach Flüchtigkeit der Ausgangsverbindung bei vermindertem oder erhöhtem Druck s.o. durchgeführt.

Schwerflüchtige oder feste Edukte II bzw. III werden in gelöster Form z.B. in Tetrahydrofuran-, Toluol- und/oder Petrolether-Lösung zum Einsatz gebracht. Generell ist eine Verdünnung der Edukte II bzw. III mit derartigen Lösungsmitteln oder mit Inertgasen wie N₂, Ar oder H₂O-Dampf möglich.

Nach der Umsetzung werden die entstandenen Produkte durch übliche Techniken z. B. durch Destillation aus dem Reaktionsgemisch isoliert; nichtumgesetztes Einsatzgemisch wird gegebenenfalls in die erfindungsgemäße Umsetzung zurückgeführt.

In einer besonders bevorzugte Ausführungsform werden die gasförmigen Reaktionsprodukte direkt (sofort) nach Verlassen des Reaktors in eine Trennung eingebracht und anschließend in ihre Einzelkomponenten zerlegt werden. Eine derartige Trennung kann z.B. in einer Fraktionierkolonne durchgeführt werden. Dies ist empfehlenswert, um eine Rückreaktion zu unterbinden und um einen hohen Umsatz zu erzielen.

Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1 : 2 (siehe Ullmanns Encyclopädie d. techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, Ti, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Aufl., Bd. 24, S. 575 (1983)). So bilden bei der Mordernit-Gruppe Ketten oder bei der Chabazit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y. Für das erfindungsgemäße Verfahren in Betracht kommende Katalysatoren sind Zeolithe aus der Mordenit-Gruppe oder engporige Zeolithe vom Erionit- bzw. Chabazit-Typ oder Zeolithe vom Faujasit-Typ, z. B. Y- oder zeolithe sowie X-, Beta- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et al. Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-A-4,512,961.

Besonders vorteilhaft sind Zeolithe vom Pentasiltyp (MFI-Struktur; G.T. Kokotailo und W.M. Meier, Spec. Publ. Chem. Soc. 33 (1980) 133).

Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolith oder deren Gemische sowie Alumino-, Boro-, Gallium- und Eisengermanatzeolithe oder deren Gemische oder Titansilikatzeolithe wie TS-1, ETS 4 und ETS 10.

Insbesondere eignen sich die Alumino-, Boro-, Gallium- und Eisensilikatzeolithe des Pentasiltyps für das erfindungsgemäße Verfahren.

Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach EP-A-34 727 und EP-A-46 504. Die erhaltenen Aluminosilikatzeolithe enthalten je nach Wahl der Einsatzstoffmengen ein SiO₂/Al₂O₃-Verhältnis von 10 bis 40 000. Auch lassen sich derartige Aluminosilikatzeolithe in etherischem Medium wie Diethylenglykol-dimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali-oder Erdalkalizusatz zur Reaktion bringt. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Der Galliumsilikatzeolith des Pentasiltyps wird z.B. bei 90 bis 200°C unter autogenem Druck synthetisiert, indem man eine Galliumverbindung, z.B. ein Alkaligallat, bevorzugt Natriumgallat, oder ein Galliumoxid oder Galliumhalogenid oder andere geeignete Galliumsalze, mit einer Siliciumverbindung, z.B. Alkalisilikaten, Kieselsolen, Kieselsäureestern oder vorzugsweise hochdispersem Siliciumdioxid in wäßriger Aminlösung, z.B. primären, sekundären oder tertiären Aminen oder quartären Alkylammoniumverbindungen, wobei ein oder mehrere Aminfunktionen pro Molekül vorliegen können, z.B. in 1,6-Diaminohexan-Lösung oder insbesondere Tetrapropylammoniumhydroxidlösung, mit oder ohne Alkali- oder Erdalkalizusatz zur Reaktion bringt. Die Herstellung von Zeolithen in Gegenwart dieser Amine ist beispielsweise beschrieben in US-A-3,702,886, BE-A-886 833, BE-A-882 484 und DE-A-30 06 471.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen (SiO₂/Al₂O₃ > 10) gehören auch die sog. ZSM-Typen, Zeolith Beta, Ferrierit, EU-1, NU-1 und Silicalite (ein Molekularsieb, ein sog. Silica Polymorph), d.h. SiO₂-Phasen mit Pentasilstruktur, deren Kenndaten und ein Verfahren zur Herstellung, z.B. in DE-A-27 51 443 (US-A-4,061,724) sowie den EP-A-64 372, EP-A-93 476 und EP-A-123 060 beschrieben sind.

Auch die sogenannten ultrastabilen Zeolithe, z.B. des Faujasit-Typs oder nordenit-Typs, das sind dealuminierte Y-Zeolithe oder dealuminierter nordenit, deren Herstellung z.B. in der US-A-4 512 961 sowie bei H.K. Beyer und S. Belenykaja, Stud. Surf. Sci. Catal. 5 (1980) 203 bis 209 sowie bei I.M. Newsam, Science, Vol. 231 (1986) 1094 beschrieben sind, können für das erfindungsgemäße Verfahren eingesetzt werden.

Weiterhin läßt sich der Beta-Zeolith, wie z.B. in US-A-4,891,458 beschrieben, vorteilhaft für die erfindungsgemäße Reaktion einsetzen.

Ferner kommen Titansilikate mit Pentasilstruktur in Betracht, wie z.B. TS-1, die z. B. beschrieben werden von B. Kraushaar und I.H.C. van Haaff in Catalysis Letters 1 (1988) 81 bis 89 oder G. Perego et. al. in Stud. Surf. Sci. Catal. 28 (1986) 129 bis 136. Ebenso können die ETS-Molekularsiebe wie z. B. ETS-1, ETS-4 und ETS-10 (US-A-4,853,202 und ZA 88 09 457) eingesetzt werden.

Die so hergestellten Alumino-, Gallium-, Boro-, Titan- und Eisensilikatzeolithe oder Silicalite können nach ihrer Isolierung, Trocknung bei 100 bis 160°C, vorzugsweise 110°C und Calcinierung bei 450 bis 550°C, vorzugsweise 500°C, mit einem Bindemittel im Verhältnis von 90:10 bis 40:60 Gew.-% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn die isolierten Zeolithe wie der Alumino-, Borosilikatzeolith direkt nach der Trocknung verformt werden und erst nach der Verformung einer Calcinierung unterworfen werden. Die hergestellten Alumino- und Borosilikatzeolithe können auch in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren.

Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. -8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, seltene Erdmetalle wie La, Ce, Pr, Nd, Fr, Yb und U eingesetzt. Insbesondere die Elemente Pd, Pt, Rh, Ru, Re, Co, Cu, Cr, Fe, Ag, Zn, Mo und W werden für das erfindungsgemäße Verfahren eingesetzt.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit.

Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine wäßrige Ni(NO₃)₂-Lösung oder ammoniakalische Pd(NO₃)₂-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.-%igen, insbesondere 12 bis 20 gew.-%igen wäßrigen Salzsäure. Anschließend wird der so behandelte Zeolith mit Wasser gewaschen, getrocknet und bei 400 bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450 bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5 Stunden, vorzugsweise mit 12 bis 20 gew.-%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger NaH₂PO₄-Lösung getränkt, bei 110°C getrocknet und bei 500°C calciniert.

Weitere Katalysatoren für die Herstellung von bifunktionellen Bausteinen aus Dialkoxialkansäureester sind Phosphate, insbesondere Aluminiumphosphate, Siliciumaluminiumphosphate, Cerphosphat, Zirkonphosphate, Borphosphat, Eisenphosphat oder deren Gemische.

Als Aluminiumphosphat- und Siliciumaluminiumphosphat-Katalysatoren werden für das erfindungsgemäße Verfahren insbesondere unter hydrothermalen Bedingungen synthetisierte substituierte und unsubstituierte Aluminiumphosphate und Siliciumaluminiumphosphate eingesetzt.

Die den Zeolithen strukturverwandten Aluminiumphosphatkatalysatoren werden für das erfindungsgemäße Verfahren insbesondere unter hydrothermalen Bedingungen synthetisiert. Es sind Siliciumaluminophosphate (Acronym SAPO) oder Aluminophosphate (Acronym AlPO₄). Diese kristallinen Festkörper weisen definierte Hohlraum- und Porenstrukturen auf und sind mit den Zeolithen strukturverwandt.

Die Herstellung, Eigenschaften und die Klassifizierung dieser Festkörper auf der Basis von Struktur und chemischer Zusammensetzung werden ausführlich von R.M. Barrer, Pure and App. Chem., Vol. 58, No. 10, pp. 1317 bis 1322, (1986); von E.M. Flanigen et al, Pure and Appl. Chem., Vol. 58, No. 10, pp. 1351 bis 1358 (1986), oder von N.B. Milestone et al, Stud. Surf. Sci. Catal. (1988), 36 (Methane Convers.), pp. 553 bis 562 beschrieben.

Aus den derzeit bekannten Kristallstrukturen und den zahlreichen Elementmodifizierungen sind inzwischen gut 700 verschiedene Kombinationen möglich. Die Bezeichnung der Aluminophosphate erfolgt durch folgende Acronyme AlPO₄, SAPO, MeAPO, MeAPSO, ElAPO oder ElAPSO. Dabei bedeuten A oder Al Aluminium, S Silicium, P steht für Phosphor und O für Sauerstoff. Unter Me sind Metalle von Fe, Mg, Mn, Co und Zn und unter El Elemente wie Be, Ga, Ge, Ti, As, B oder Li zu verstehen. Eine mit Bindestrich angefügte Zahl dient der Kennzeichnung der Kristallstruktur der betreffenden Phase.

Die unter hydrothermalen Bedingungen hergestellten Aluminiumphosphate sind z.B. AlPO-5, AlPO-9, AlPO-11, AlPO-12, AlPO-14, AlPO-21, AlPO-25, AlPO-31, AlPO-33, AlPO-34, AlPO-37 und AlPO-54. Synthesen dieser Verbindungen sind in EP-A-132 708, US-A-4,310,440 und US-A-4,473,663 beschrieben. Eine Übersicht über diese Verbindungsklasse ist gegeben in Pure and Applied Chemistry Vol 58, 10 (1986) 1351 bis 1358.

Beispielsweise das AlPO₄-5 (APO-5) wird synthetisiert, indem man Orthophosphorsäure mit Pseudoboehmit (Catapal® SB) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte AlPO₄ wird getrocknet bei 100 bis 160°C und calciniert bei 450 bis 550°C.

AlPO₄-9 (APO-9) wird ebenfalls aus Orthophosphorsäure und Pseudoboehmit aber in wäßriger DABCO-Lösung (1,4-Diazabicyclo-(2,2,2)-octan) bei ca. 200°C unter autogenem Druck während 200 bis 400 h synthetisiert.

Die Synthese des AlPO₄-21 (APO-21) erfolgt aus Orthophosphorsäure und Pseudoboehmit in wäßriger Pyrrolidon-Lösung bei 150 bis 200°C unter autogenem Druck während 50 bis 200 h.

Die Synthesen für MeAPO's sind US-A-4,544,143, EP-A-132 708 und US-A-4,567,029 sowie für ELAPO in US-A-4,500,651 und EP-A-158 976 beschrieben.

Für das erfindungsgemäße Verfahren bevorzugt sind insbesondere die Silicium-haltigen Aluminophosphate (SAPO, MeAPSO oder ElAPSO) wie SAPO-11, SAPO-5, SAPO-20, SAPO-34, SAPO-37, SAPO-41 oder SAPO-46.

Synthesen der Silicium-aluminophosphate sind u.a. beschrieben für SAPO in US-A-4,440,871 und EP-A-103 117, für MeAPSO in EP-A-158 348, EP-A-158 975 und EP-A-161 491, und für ElAPSO in EP-A-159 624.

SAPO's werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

Als Siliciumaluminiumphosphate sind z.B. ZYT-5, ZYT-6, ZYT-7, ZYT-9, ZYT1-11 und ZYT-12 geeignet (JP 59 217-619).

SAPO-5 beispielsweise wird durch Mischen von SiO₂ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x H₂O in 700 ml Wasser über 2 h zugetropft. Dabei wird der pH-Wert durch gleichzeitige Zugabe von 25%iger NH₃-Lösung bei pH 8 gehalten. Der entstandene Niederschlag wird 12 h nachgerührt, dann abgesaugt und ausgewaschen. Er wird bei 60°C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C herstellen.

CePO₄ wird durch Fällung aus 52 g Ce(NO₃)₃ x 6 H₂O und 56 g NaH₂PO₄ x 2 H₂O erhalten. Nach der Filtration wird das Material zu Strängen verformt, bei 120°C getrocknet und bei 450°C calciniert. Der Katalysator enthält 47,1 Gew.-% Ce und 12,7 Gew.-% P.

Als Zirkonphosphate kommen im Handel erhältliche Zirkonphosphate wie z.B. CSZ 100, Zirkonphosphatsilikate und Zirkonphosphate, die NH₃ adsorbieren bzw. adsorbiert haben, in Betracht.

Diese Phosphate bzw. Phosphate mit Zeolithstruktur können, wie zuvor bei den Zeolithen gezeigt, modifiziert werden durch Dotierung mit Metallen, Säurebehandlung, Steamen etc.

Die hier beschriebenen Katalysatoren können wahlweise als 2- bis 4-mm Stränge oder als Tabletten mit 3 bis 5 mm Durchmesser oder als Splitt mit Teilchengrößen von 0,1 bis 0,5 mm oder als Wirbelkontakt eingesetzt werden.

Die Substituenten R¹ und R² sowie der Index n in den Verbindungen der allgemeinen Formeln I, II und III haben die folgenden Bedeutungen:
- R¹, R²: - unabhängig voneinander
- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt Cl- bis C4-Alkyl wie Methyl, Ethyl, n-Propyl, iso-Propyl, n-Butyl, iso-Butyl, sec.-Butyl und tert.-Butyl,
- C₁- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Alkyl-cycloalkyl, bevorzugt C₆- bis C₂₀-Alkyl-cycloalkyl wie 2-Methyl-cyclopentyl, 3-Methylcyclohexyl und 4-Methylcyclohexyl,
- C₄- bis C₂₀-Cycloalkyl-alkyl, bevorzugt C₆- bis C₂₀-Cycloalky-alkyl wie Cyclopentyl-methyl, Cyclohexyl-methyl und Cyclohexyl-ethyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethyl-phenyl, 2,5-Dimethyl-phenyl, 2,6-Dimethylphenyl, 3,4-Dimethyl-phenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇- bis C₂₀-Aralkyl, bevorzugt C₇- bis C₁₂-Phenyl-alkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- einen heterocyclischen Rest wie einen aromatischen und einen nicht-aromatischen Heterocyclus mit ein bis drei Heteroatomen wie Stickstoff, Sauerstoff und/oder Schwefel, bevorzugt Stickstoff und Sauerstoff,
- n: - eine ganze Zahl von 3 bis 7 wie 3, 4, 5, 6 und 7, bevorzugt 4 und 5.

Als Ketone II seien vorzugsweise genannt:
Acetophenon, Benzophenon, Phenylaceton, Methyl-isopropylketon, Butanon-2, Pentanon-2, Pentanon-3, Hexanone, Cyclohexanon und Cyclopentanon.

Als cyclische Amine III seien vorzugsweise genannt:
Pyrrolidin, Morpholin und Piperidin.

Die Herstellung derartiger Ausgangsstoffe der Formeln II und III ist hinlänglich aus Standardwerken (Beilstein, Gmelin) bekannt.

Die Amine I sind in der Regel wertvolle Bausteine in der organischen Synthese. Besonders interessant sind solche Verbindungen, als Vorprodukte für Pharmazeutika und Wirkstoffe in Herbiziden, Fungiziden sowie Insektiziden und als Katalysatoren für die organische Synthese bzw. bei Polymerisationen.

Die folgenden Beispiele veranschaulichen die Erfindung.

### Gasphasenreaktion

Die Reaktionen in der Gasphase wurden unter isothermen Bedingungen in einem Rohrreaktor (Wendel, 0,6 cm Innendurchmesser, 90 cm Länge) an einem Katalysatorfestbett durchgeführt. Dabei wurde die Katalysatormenge zwischen 1 und 20 g, entsprechend einer Belastung WHSV zwischen von 0,1 und 10/h variiert. Die Reaktionsprodukte wurden durch übliche Methoden abgetrennt und durch GC/MS charakterisiert. Die quantitative Bestimmung der Reaktionsprodukte und der Ausgangsprodukte erfolgte gaschromatographisch oder durch Auswiegen der durch Destillation oder Extraktion erhaltenen Fraktionen.

Die für das erfindungsgemäße Verfahren eingesetzten Katalysatoren sind:

### Katalysator A

Der Borosilikatzeolith des Pentasil-Typs wird in einer hydrothermalen Synthese aus 640 g hochdispersem SiO₂, 122 g H₃BO₃, 8 000 g einer wäßrigen 1,6-Hexandiamin-Lösung (Mischung 50:50 Gew.-%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert.

Dieser Borosilikatzeolith setzt sich zusammen aus 94,2 Gew.-% SiO₂ und 2,3 Gew.-% B₂O₃.

Mit diesem Material werden durch Verformen mit Boehmit als Binder (Gewichtsverhältnis 60:40) 2-mm-Stränge hergestellt, die bei 110°C/16h getrocknet und bei 500°C/24 h calciniert werden.

Katalysator A wird erhalten, indem man diese Stränge einem Ionenaustausch mit ammoniakalischer Palladiumnitrat-Lösung unterwirft und dann bei 110°C/16 h trocknet und bei 500°C/5 h calciniert. Der Pd-Gehalt beträgt 3,3 Gew.-%.

### Katalysator B

Der mit Boehmit verformte Borosilikatzeolith des Pentasil-Typs (siehe Katalysator A) wird mit einer wäßrigen AgNO₃-Lösung imprägniert. Danach wird bei 130°C/2 h getrocknet und bei 540°C/2 h calciniert.

### Katalysator C

Der mit Beohmit verformte Borosilikatzeolith des Pentasil-Typs (siehe Katalysator A) wird mit einer 20 %igen wäßrigen NH₄Cl-Lösung bei 80°C/2 h behandelt. Danach wird Cl-frei gewaschen. Nunmehr wird mit einer ammoniakalischen Pd-Nitrat-Lösung bei 50°C in einer Kreislaufapparatur 16 h behandelt. Nach Trocknung bei 110°C/2 h und Calcination bei 500°C/5 h beträgt der Pd-Gehalt 0,34 Gew.-%.

### Katalysator D

Borosilikatzeolith-Pulver wird wie bei Katalysator A beschrieben hergestellt und bei 50°C/4 h mit einer ammoniakalischen Pd-Nitratlösung und einer NaNO3-Lösung einem Ionenaustausch unterworfen. Danach wird bei 110°C/2 h getrocknet und bei 500°C/5 h calciniert. Der Pd-Gehalt des ionenausgetauschten Zeolithen beträgt 0,45 Gew.-% und der Na-Gehalt 0,12 Gew.-%. Dieses Pulver wird mit Verformungshilfsmittel zu 2,5-mm-Strängen bei einem Preßdruck von 110 bar verformt. Die Stränge werden bei 100°C/2 h getrocknet und bei 500°C/16 h calciniert.

### Katalysator E

Der mit Boehmit verformte Borositkatzeolith des Pentasil-Typs (siehe Katalysator A) wird mit einer ammoniakalischen Pt-Chlorid-Lösung imprägniert. Danach bei 130°C/2 h getrocknet und bei 540°C/2 h calciniert. Der Pt-Gehalt beträgt danach 2,49 Gew.-%.

### Katalysator F

Borosilikatzeolith-Pulver wird wie bei Katalysator A beschrieben hergestellt und bei 50°C/4 h mit einer ammoniakalischen Pt-Chlorid-Lösung ionenausgetauscht. Nach Abfiltrieren und Auswaschen mit H₂O wird bei 110°C/2 h getrocknet und bei 500°C/5 h calciniert. Der Pt-Gehalt beträgt danach 2,11 Gew.-%.

### Katalysator G

Ein Aluminosilikatzeolith vom Pentasil-Typ wurde unter hydrothermalen Bedingungen bei autogenem Druck und 150°C aus 65 g hochdispersem SiO₂, 20,3 g Al₂(SO₄)₃ x 18 H₂O in 1 kg einer wäßrigen 1,6-Hexandiamin-Lösung (Mischung 50 : 50 Gew.-%) in einem Rührautoklaven hergestellt. Nach Abfiltrieren und Auswaschen wurde das kristalline Reaktionsprodukt bei 110°C/24 h getrocknet und bei 500°C/24 h calciniert. Dieser Aluminosilikatzeolith enthielt 91,6 Gew.-% SiO₂ und 4,6 Gew.-% Al₂O₃. Der Katalysator wurde mit einem Verformungshilfsmittel zu 2-mm-Strängen verformt, bei 110°C/16 h getrocknet und bei 500°C/24 h calciniert.

Diese Stränge werden mit einer wäßrigen Lösung aus Cu-Nitrat, Ni-Nitrat und Pd-Chlorid imprägniert. Nach Trocknung bei 130°C/2 h und Calcination bei 540°C/2 h betragen der Cu-Gehalt 2,6 Gew.-%, der Ni-Gehalt 0,57 Gew.-%, der Pd-Gehalt 0,05 Gew.-% und der Cl-Gehalt 0,06 Gew.-%.

### Katalysator H

Kommerziell erhältlicher Na-Y-Zeolith wird mit Boehmit im Gewichtsverhältnis 60:40 zu 2-mm-Strängen bei einem Preßdruck von 100 bar verformt, bei 110°C/2 h getrocknet und bei 500°C/16 h calciniert. Diese Stränge werden mit einer ammoniakalischen Pd-Nitrat-Lösung bei 50°C ionenausgetauscht. Nach Trocknung bei 110°C/2 h und Calcination bei 500°C/5 h betragen der Pd-Gehalt 2,33 Gew.-% und Na-Gehalt 3,6 Gew.-%.

### Katalysator I

Na-Y-Zeolithstränge - wie bei Katalysator H beschrieben - werden mit einer 20 % NH₄Cl-Lösung bei 80°C/2 h ionenausgetauscht. Dieser Austausch wird 3 mal wiederholt. Nach Trocknung bei 110°C/2 h und Calcination bei 500°C/5 h beträgt der Na-Gehalt 0,2 Gew.-%. Nunmehr wird mit einer wäßrigen Co-Nitrat-Lösung bei 80°C/2 h ionenausgetauscht. Nach Auswaschen mit H₂O wird bei 130°C/2 h getrocknet und bei 540°C/2 h calciniert.

### Katalysator J

Der bei Katalysator J durch Ionenaustausch hergestellte HY-Zeolith (allerdings ohne Boehmit-Verformung) wird mit einer wäßrigen Cu-Nitrat-Lösung behandelt, mit einem Verformungshilfsmittel zu 2-mm-Strängen verformt, bei 130°C/2 h getrocknet und bei 540°C/2 h calciniert. Der Cu-Gehalt beträgt 5 Gew.-%.

### Katalysator K

Kommerziell bei Union Carbid erhältliches 13X-Molsieb mit einem SiO₂-Gehalt von 46,5 Gew.-%, Al₂O₃-Gehalt von 27,9 Gew.-% und Na-Gehalt von 11,3 Gew.-% wird in einer Kolonne mit 20%iger wäßriger Cu-Nitrat-Lösung bei 80°C/2 h ionenausgetauscht. Danach wird NO₃-frei gewaschen, bei 110°C/2 h getrocknet und bei 500°C/5 h calciniert. Der Cu-Gehalt beträgt 13,9 Gew.-%, der Na-Gehalt 1,3 Gew.-%.

### Katalysator L

Kommerziell bei PQ-Corporation erhältlicher Beta-Zeolith mit einem SiO₂-Gehalt von 87,0 Gew.-% und einem Al₂O₃-Gehalt von 4,9 Gew.-% wird mit einer ammoniakalischen Pd-Nitrat-Lösung bei 50°C/4 h ionenausgetauscht. Nach Abfiltrieren wird bei 110 °C/2 h getrocknet und bei 500°C/5 h calclniert. Der Pd-Gehalt beträgt 2,73 Gew.-%. Dieses Pulver wird mit Verformungshilfsmitteln zu 2-mm-Strängen verformt, bei 110°C getrocknet und bei 500°C/16 h calciniert.

### Katalysator M

AlPO₄-5 (APO-5) wird synthetisiert, indem man 200 g 98%ige Phosphorsäure und 136 g Boehmit in 335 g Wasser löst bzw. suspendiert, hierzu 678 g einer 30%igen wäßrigen Tetrapropylammoniumhydroxid-Lösung zugibt und diese Mischung in einem Rührautoklaven bei 150°C in 43 h unter autogenem Druck umsetzt. Nach Abfiltrieren wird das kristalline Material bei 120°C getrocknet und 500°C/16 h calciniert. Das so synthetisierte AlPO₄-5 enthält 45,5 Gew.-% Al₂O₃ und 46,5 Gew.-% P₂O₅. Dieses Material wird mit einer wäßrigen Rh-Nitrat-Lösung behandelt und mit Verformungshilfsmittel zu 2-mm-Strängen verformt, abermals bei 120°C getrocknet und bei 500°C/16 h calciniert. Der Rh-Gehalt beträgt 2 Gew.-%.

### Katalysator N

Siliciumaluminiumphosphat-5 (SAPO-5) wird hergestellt aus einer Mischung aus 200 g 98%ige Phosphorsäure, 136 g Boehmit, 60 g Kieselsol (30%ig) 287 g Tripropylamin und 587 g H₂O. Diese Mischung wird bei 150°C während 168 Std. unter autogenem Druck umgesetzt. Nach Filtration wird das kristalline Produkt bei 120°C getrocknet und bei 500°C calciniert. SAPO-5 enthält 49,8 Gew.-% P₂O₅, 33,0 Gew.-% Al₂O₃, 6,2 Gew.-% SiO₂. SAPO-5 wird mit hochdispersen Kieselgel im Gewichtsverhältnis 80:20 zu 2-mm-Strängen verformt, bei 110°C getrocknet und bei 500°C/16 h calciniert. Diese Stränge werden mit ammoniakalischer Pd-Nitrat-Lösung bei 50°C ionenausgetauscht. Nach Trocknung bei 110°C/2 h und Calcination bei 500°C /16 h beträgt der Pd-Gehalt 0,5 Gew.-%.

### Katalysator O

Ein SAPO-11 wird synthetisiert, indem man 200 g Orthophosphorsäure (98 Gew.-%) mit 417 g Aluminiumtriisopropylat und 60 g Kieselsol (30 Gew.-% SiO₂) in 927 g Wasser homogen mischt; zu dieser Mischung werden 91,5 g Di-n-propylamin hinzugefügt. Danach wird bei 200°C 96 h unter autogenem Druck in einem Rühr-Autoklaven umgesetzt. Das abfiltrierte und gewaschene Siliciumaluminiumphosphat wird getrocknet bei 110°C und calciniert bei 500°C. Die Zusammensetzung des SAPO-11 ist 40,4 Gew.-% Al₂O₃, 49,5 Gew.-% P₂O₅ und 1,87 Gew.-% SiO₂. Mit diesem Material werden durch Verformen mit einem Verformungshilfsmittel 2-mm-Stränge hergestellt, die bei 110°C/16 h getrocknet und bei 500°C/16 h calciniert werden. Diese Stränge wurden mit einer ammoniakalischen Pd-Nitrat-Lösung bei 50°C ionenausgetauscht. Nach Trocknung bei 110°C/2 h und Calcination bei 500°C/16 h beträgt der Pd-Gehalt 0,5 Gew.-%.

### Katalysator P

Kommerziell bei Union Carbide Corporation erhältlicher SAPO-34 wird mit pyrogenem Kieselgel im Gewichtsverhältnis 80:20 zu 2-mm-Strängen verformt, bei 110°C getrocknet und bei 500°C/16 h calciniert. Diese Stränge werden mit einer ammoniakalischen Pd-Nitrat-Lösung bei 50°C ionenausgetauscht. Nach Filtrieren wird bei 110°C/2 h getrocknet und bei 500°C/16 h calciniert. Der Pd-Gehalt beträgt 0,5 Gew.-%.

Die mit diesen Katalysatoren erhaltenen Versuchsergebnisse sind in den nachfolgenden Tabellen aufgelistet.
- Reaktion A:: Benzophenon + Pyrrolidin →
- Reaktion B:: Methylisopropylketon + Morpholin →
- Reaktion C:: Methylisopropylketon + Piperidin →
- Reaktion D:: Methylisopropylketon + Pyrrolidin →
- Reaktion E:: Pentanon-2 + Morpholin →
- Reaktion F:: Pentanon-2 + Piperidin →
- Reaktion G:: Pentanon-2 + Pyrrolidin →
- Reaktion H:: Pentanon-2 + 2,6-Dimethylpiperidin →
- Reaktion I:: Pentanon-2 + 4-Methylpiperidin →

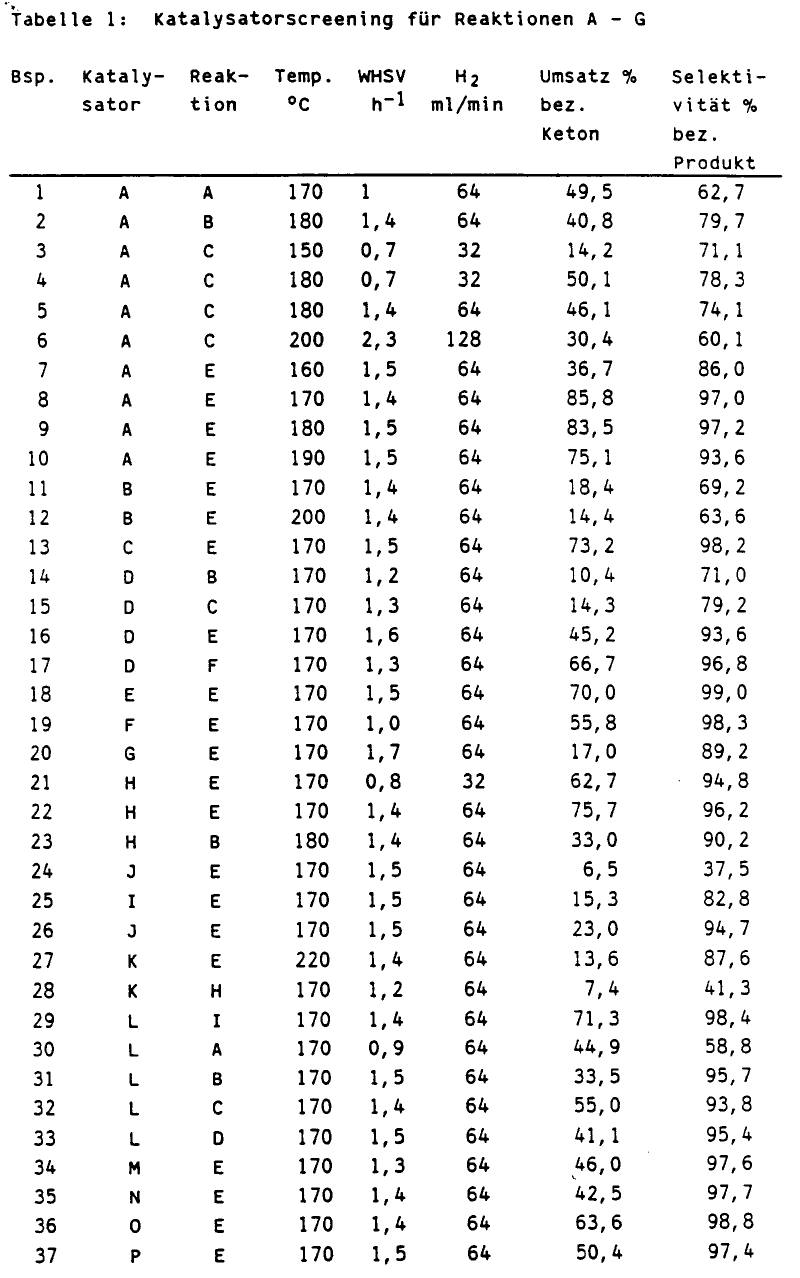

**Tabelle 2**

| Katalysator L, Reaktion F, 170°C, WHSV = 1,4 h⁻¹ 64 ml/min H₂ Beispiel 38 | | |
|---|---|---|
| Zeit [h] | Selektivitäten | |
| | Umsatz Edukt 1 | Produkt |
| 1,25 | 88,42 | 97,88 |
| 2,25 | 89,77 | 99,63 |
| 4,25 | 89,01 | 99,50 |
| 8,25 | 86,18 | 98,80 |
| 12,25 | 85,31 | 98,22 |
| 16,25 | 84,35 | 98,73 |
| 20,25 | 83,85 | 98,57 |
| 24,25 | 83,53 | 99,00 |
| 28,25 | 82,07 | 98,70 |
| 32,25 | 81,88 | 98,11 |
| 36,25 | 81,46 | 98,09 |
| 40,25 | 81,72 | 97,95 |
| 44,25 | 81,58 | 98,24 |
| 48,25 | 81,29 | 97,94 |
| 52,25 | 80,12 | 98,03 |
| 56,25 | 79,98 | 98,18 |
| 60,25 | 79,83 | 98,02 |
| 48,25 | 81,29 | 98,08 |
| 60,50 | --- | --- |
| | 83,43 | 98,43 |

**Tabelle 3**

| Katalysator L, Reaktion G, 170°C, WHSV = 1,2 h⁻¹ 64 ml/min H₂ Beispiel 39 | | |
|---|---|---|
| Zeit [h] | Selektivitäten | |
| | Umsatz Edukt 1 | Produkt |
| 0,25 | | |
| 1,25 | 79,51 | 91,27 |
| 2,25 | 86,76 | 96,92 |
| 4,25 | 88,58 | 96,78 |
| 8,25 | 89,81 | 96,95 |
| 12,25 | 90,43 | 97,48 |
| 16,25 | 90,66 | 97,01 |
| 20,25 | 90,54 | 97,36 |
| 24,25 | 90,38 | 97,58 |
| 28,25 | 89,18 | 96,32 |
| 32,25 | 88,68 | 96,03 |
| 36,25 | 88,67 | 95,91 |
| 40,25 | 88,77 | 95,91 |
| 44,25 | 88,63 | 95,64 |
| 48,25 | 88,51 | 95,51 |
| 52,25 | 86,96 | 96,03 |
| 56,25 | 86,70 | 96,80 |
| 60,25 | 86,68 | 96,54 |
| 48,25 | 88,38 | 95,49 |
| 60,25 | --- | --- |
| | 88,73 | 96,49 |

**Tabelle 4**

| Katalysator L, Reaktion G, 170°C, WHSV = 1,4 h⁻¹ 64 ml/min H₂ Beispiel 40 | | |
|---|---|---|
| Zeit [h] | Selektivitäten | |
| | Umsatz Edukt 1 | Produkt |
| 0,25 | | |
| 1,25 | 90,39 | 98,87 |
| 2,25 | 92,30 | 97,00 |
| 4,25 | 88,75 | 99,30 |
| 8,25 | 87,64 | 97,63 |
| 12,25 | 86,81 | 99,52 |
| 16,25 | 84,92 | 99,25 |
| 20,25 | 85,79 | 99,75 |
| 24,25 | 85,38 | 100,00 |
| 28,25 | 84,80 | 98,52 |
| 32,25 | 84,68 | 98,51 |
| 36,25 | 67,90 | 81,82 |
| 40,25 | 84,42 | 98,50 |
| 44,25 | 84,18 | 97,99 |
| 48,25 | 83,76 | 98,49 |
| 52,25 | 83,72 | 98,60 |
| 56,25 | 83,76 | 98,47 |
| 60,25 | 83,40 | 98,33 |
| 48,25 | 83,65 | 98,49 |
| 60,25 | --- | --- |
| | 85,49 | 98,62 |

## Patentansprüche

1. Verfahren zur Herstellung von Aminen der allgemeinen Formel I 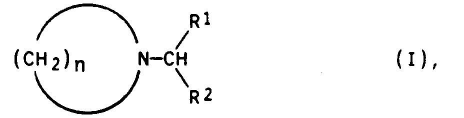 in der
R¹, R² C₁- bis C₂₀-Alkyl, C₃- bis C₂₀-Cycloalkyl, C₄- bis C₂₀-Alkyl-cycloalkyl, C₄- bis C₂₀-Cycloalkylalkyl, Aryl, C₇- bis C₂₀-Alkylaryl, C₇- bis C₂₀-Aralkyl und heterocyclische Reste und
n eine ganze Zahl von 3 bis 7
bedeuten, dadurch gekennzeichnet, daß man Ketone der allgemeinen Formel II 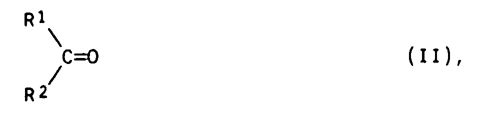 in der R¹ und R² die oben genannten Bedeutungen haben, mit einem cyclischen Amin der allgemeinen Formel III 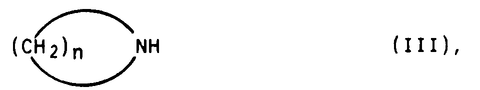 in der n die oben genannten Bedeutungen hat, wit Wasserstoff in Gegenwart von Zeolithen und/oder SiO₂ mit Zeolithstruktur und/oder Phosphaten und/oder Phosphaten mit Zeolithstruktur als Katalysatoren bei Temperaturen von 50 bis 500°C und Drücken von 0,01 bis 50 bar umsetzt.

2. Verfahren nach Anspruch 1, dadurch gekennzeichnet, daß man als Katalysatoren Zeolithe des Pentasil-, Faujasit-, Mordenit- und/oder Beta-Typs verwendet.

3. Verfahren nach Anspruch 1, dadurch gekennzeichnet, daß man als Katalysatoren Aluminium-, Gallium-, Boro- und/oder Eisensilikatzeolithe verwendet.

4. Verfahren nach Anspruch 1, dadurch gekennzeichnet, daß man mit Edelmetallen, Alkalimetallen, Übergangsmetallen, seltenen Erdmetallen dotierte Zeolithe als Katalysatoren verwendet.

5. Verfahren nach Anspruch 1 - 4, dadurch gekennzeichnet, daß man den unverformten oder verformten Zeolithen mit Pd, Pt, Rh, Re, Ru, Co, Cu, Cr, Fe, Ag, Zn als Metalle durch einen Ionenaustausch oder durch Imprägnierung dotiert.

6. Verfahren nach Anspruch 1, dadurch gekennzeichnet, daß man als Katalysatoren Phosphate der Elemente B, Al, Zr, Fe, Sr oder deren Gemische verwendet.

7. Verfahren nach Anspruch 1, dadurch gekennzeichnet, daß man als Katalysatoren hydrothermal hergestellte Phosphate, die Zeolithstruktur besitzen, verwendet.

8. Verfahren nach Anspruch 1, dadurch gekennzeichnet, daß man als Katalysatoren hydrothermal hergestellte Aluminiumphosphate oder Siliciumaluminiumphosphate mit Zeolithstruktur, die gegebenenfalls zusätzlich die Elemente Lithium, Beryllium, Bor, Magnesium, Gallium, Germanium, Arsen, Titan, Mangan, Eisen, Kobalt und/oder Zink enthalten, verwendet.

9. Verfahren nach Anspruch 1, dadurch gekennzeichnet, daß die hydrothermal hergestellten Phosphate ausgewählt sind aus der Gruppe AlPO₄, SAPO, ELAPO, ELAPSO, MeAPO oder MeAPSO.

10. Verfahren nach Anspruch 1, dadurch gekennzeichnet, daß man die Reaktion in der Gasphase durchführt.

## Claims

1. A process for the preparation of an amine of the formula I 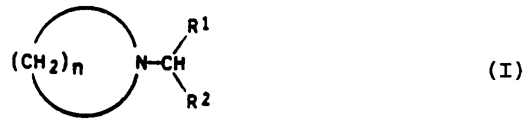 where R¹ and R² are each C₁-C₂₀-alkyl, C₃-C₂₀-cycloalkyl, C₄-C₂₀-alkylcycloalkyl, C₄-C₂₀-cycloalkylalkyl, aryl, C₇-C₂₀-alkylaryl, C₇-C₂₀-aralkyl or a heterocyclic radical and n is an integer of from 3 to 7, wherein a ketone of the formula II 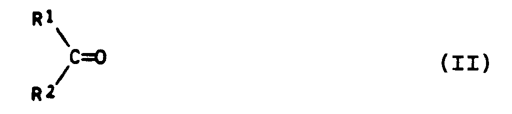 where R¹ and R² have the abovementioned meanings, is reacted with a cyclic amine of the formula III 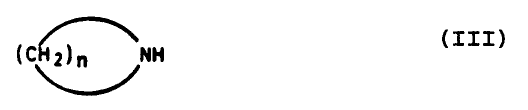 where n has the abovementioned meanings, together with hydrogen in the presence of a zeolite or SiO₂ having a zeolite structure or a phosphate or a phosphate having a zeolite structure as a catalyst at from 50 to 500°C and from 0.01 to 50 bar.

2. A process as claimed in claim 1, wherein the catalyst used is a zeolite of the pentasil, faujasite, mordenite or beta type.

3. A process as claimed in claim 1, wherein the catalyst used is an aluminosilicate, gallosilicate, borosilicate or ferrosilicate zeolite.

4. A process as claimed in claim 1, wherein the catalyst used is a zeolite doped with noble metals, alkali metals, transition metals or rare earth metals.

5. A process as claimed in claim 4, wherein the unmolded or molded zeolite is doped with the metals Pd, Pt, Rh, Re, Ru, Co, Cu, Cr, Fe, Ag and Zn, by ion exchange or by impregnation.

6. A process as claimed in claim 1, wherein the catalyst used is a phosphate of the element B, Al, Zr, Fe or Sr or of a mixture thereof.

7. A process as claimed in claim 1, wherein the catalyst used is a hydrothermally prepared phosphate which has a zeolite structure.

8. A process as claimed in claim 1, wherein the catalyst used is a hydrothermally prepared aluminum phosphate or silicon aluminophosphate having a zeolite structure, which may additionally contain the elements lithium, beryllium, boron, magnesium, gallium, germanium, arsenic, titanium, manganese, iron, cobalt or zinc.

9. A process as claimed in claim 1, wherein the hydrothermally prepared phosphate is selected from the group consisting of AlPO₄, SAPO, ElAPO, ElAPSO, MeAPO or MeAPSO.

10. A process as claimed in claim 1, wherein the reaction is carried out in the gas phase.

## Revendications

1. Procedé de préparation d'amines de la formule générale I 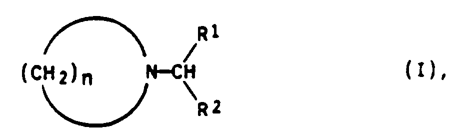 dans laquelle
R¹, R² représentent des radicaux alkyle en C₁ à C₂₀, cycloalkyle en C₃ à C₂₀, alkylcycloalkyle en C₄ à C₂₀, cycloalkylalkyle en C₄ à C₂₀, aryle, alkylaryle en C₇ à C₂₀, aralkyle en C₇ à C₂₀ et des radicaux hétérocycliques et
n représente un nombre entier dont la valeur varie de 3 à 7
caractérisé en ce que l'on fait réagir des cétones de la formule générale II 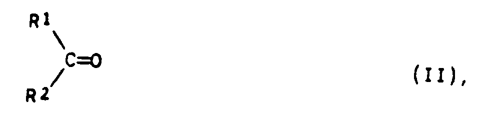 dans laquelle R¹ et R² possèdent les significations qui leur ont été attribuées ci-dessus, avec une amine cyclique de la formule générale III 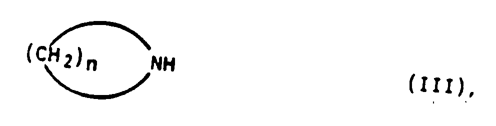 dans laquelle n possède les significations qui lui ont été attribuées ci-dessus, avec de l'hydrogène, en présence de zéolites et/ou de SiO₂ à structure zéolitique et/ou des phosphates et/ou des phosphates à structure zéolitique, à titre de catalyseurs, à des températures de 50 à 500°C et sous des pressions de 0,01 à 50 bars.

2. Procédé suivant la revendication 1, caractérisé en ce que l'on utilise, à titre de catalyseurs, des zéolites du type pentasile, faujasite, mordénite et/ou du type bêta.

3. Procédé suivant la revendication 1, caractérisé en ce que l'on utilise, à titre de catalyseurs, des zéolites à base de silicates d'aluminium, de gallium, de bore et/ou de fer.

4. Procédé suivant la revendication 1, caractérisé en ce que l'on utilise, à titre de catalyseurs, des zéolites dopées de métaux nobles, de métaux alcalins, de métaux de transition, de métaux des terres rares.

5. Procédé suivant les revendications 1-4, caractérisé en ce que l'on dope les zéolites non transformées ou transformées avec Pd, Pt, Rh, Re, Ru, Co, Cu, Cr, Fe, Ag, Zn à titre de métaux par l'intermédiaire d'un échange d'ions, ou par imprégnation.

6. Procédé suivant la revendication 1, caractérisé en ce que l'on utilise, à titre de catalyseurs, des phosphates des éléments B, Al, Zr, Fe, Sr, ou leurs mélanges.

7. Procédé suivant la revendication 1, caractérisé en ce que l'on utilise, à titre de catalyseurs, des phosphates préparés par voie hydrothermique, qui possèdent une structure zéolitique.

8. Procédé suivant la revendication 1, caractérisé en ce que l'on utilise, à titre de catalyseurs, des phosphates d'aluminium préparés par voie hydrothermique, ou des phosphates de silicium-aluminium à structure zéolitique, qui contiennent éventuellement complémentairement les éléments lithium, béryllium, bore, magnésium, gallium, germanium, arsenic, titane, manganèse, fer, cobalt et/ou zinc.

9. Procédé suivant la revendication 1, caractérisé en ce que les phosphates préparés par voie hydrothermique sont choisis dans le groupe formé par les suivants : AlPO₄, SAPO, ELAPO, ELAPSO, MeAPO ou MeAPSO.

10. Procédé suivant la revendication 1, caractérisé en ce que l'on entreprend la réaction en phase gazeuse.
